(19) Europäisches Patentamt
European Patent Office
Office européen des brevets

(11) Publication number : **0 283 166 B1**

(12) # EUROPEAN PATENT SPECIFICATION

(45) Date of publication of patent specification : **02.01.92 Bulletin 92/01**

(21) Application number : **88301822.8**

(22) Date of filing : **02.03.88**

(51) Int. Cl.$^5$ : **C07D 249/20,** C08F 20/12, C08F 20/28, C08F 20/36, C08L 33/12, C08L 3/14, G02B 1/04, G02C 7/04, A61F 2/16

(54) **2-(2'-Hydroxyphenyl)-5(6) (acryloyloxyalkoxy)-benzotriazole and ultraviolet absorbing polymers therefrom.**

(30) Priority : **03.03.87 US 21096**

(43) Date of publication of application : **21.09.88 Bulletin 88/38**

(45) Publication of the grant of the patent : **02.01.92 Bulletin 92/01**

(84) Designated Contracting States : **DE FR GB IT**

(56) References cited :
**EP-A- 0 131 468**
**EP-A- 0 133 164**
**FR-A- 2 096 034**
**US-A- 3 159 646**
**US-A- 3 399 173**

(73) Proprietor : **IOLAB CORPORATION**
**500 Iolab Drive**
**Claremont, California 91711-4881 (US)**

(72) Inventor : **Dunks, Gary B.**
**1100 Emerson Street**
**Upland, CA 917786 (US)**
Inventor : **Yamada, Akira**
**261 Ferris Street**
**Claremont, CA 91711 (US)**
Inventor : **Beard, Charles D.**
**55 Guyencourt Road, Box 560,**
**Montchanin, DE 19710 (US)**
Inventor : **Doddi, Namassivaya**
**1658 Erin Avenue**
**Upland, CA 91786 (US)**

(74) Representative : **Jones, Alan John et al**
**CARPMAELS & RANSFORD 43 Bloomsbury Square**
**London, WC1A 2RA (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 283 166 B1

Jouve, 18, rue Saint-Denis, 75001 PARIS

## Description

The invention relates to novel 2-(2′-hydroxyphenyl)-5(6)-(acryloyloxyalkoxy)benzotriazoles, to ultraviolet absorbing polymers produced therefrom, and to ocular devices, particularly intraocular lenses and contact lenses, prepared from such polymers.

The absorption of radiation in the ultraviolet range by polymeric materials is a major cause of the light-induced degradation therein. It is standard practice to add a low molecular weight UV "stabilizer" to light-sensitive polymers to absorb the light in the destructive range or to quench the energy generated as a result of the excitation of the light-absorbing functional groups in the polymer.

Although low molecular weight UV absorbers or quenchers of various types are effective in inhibiting or retarding the destruction of the polymers to which they are added, their extractability in various media and/or their volatility during the processing or fabrication of the polymers at elevated temperatures place a limitation on their utility.

This problem has been remedied to a considerable extent by the synthesis of copolymerizable monomers containing structural moieties capable of functioning as UV absorbers or quenchers. The copolymerization of such monomers results in the formation of copolymers with increased stability, i.e. resistance to degradation upon exposure to UV light, with decreased extractability and volatility. The addition of such polymers to a suitable matrix polymer imparts these properties to the latter.

US-A-4,304,895 discloses the use of 2-hydroxy-4-methacryloyloxybenzophenone and mixtures thereof as a monomeric ultraviolet light absorber copolymerizable with acrylic monomers and useful in the preparation of UV absorbing hard contact lenses.

Similarly, the copolymerization of an allyl-2-hydroxy-benzophenone with an acrylate ester such as methyl methacrylate is described in US-A-4,310,650, and the copolymerization of ethylenically unsaturated derivatives of 2,4-dihydroxy-benzophenone with other vinyl type comonomers is broadly disclosed in US-A-3,162,676.

Copolymers of methyl methacrylate and 4-(beta-acryloyloxyethoxy)-2-hydroxybenzophenone have been used as UV-absorbing intraocular lenses.

US-A-4,528,311 discloses certain benzotriazole monomers which are copolymerizable with vinyl monomers such as methyl methacrylate to yield optically clear polymers useful in the preparation of intraocular and contact lenses. Representative of the disclosed benzotriazole monomers and a particularly preferred compound is 2-[3′-t-butyl-2′-hydroxy-5′-(3″-methacryloyloxypropyl)phenyl]-5-chloro-2H-benzotriazole, which has the structure :

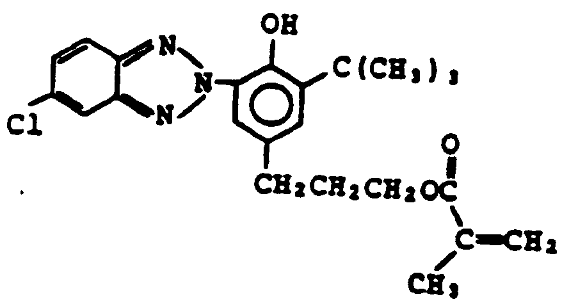

UV absorbing lenses are particularly desirable for use by persons who have had their natural lenses surgically removed owing to cataracts or other deterioration of the lens. The visual correction of aphakia resulting from such lens removal requires the use of high plus corrective lenses, which may be in the form of spectacles, contact lenses, or intraocular lenses.

In the normal eye, a portion of incident light entering the eye is absorbed by various parts of the eye so that only the unabsorbed or transmitted portion strikes the retina. Incident light may comprise the entire spectrum of wavelengths including the ultraviolet, visible and infrared.

The cornea preferentially absorbs the ultraviolet portion of the light with wavelengths up to about 300 nm (nanometers). The crystalline lens preferentially absorbs ultraviolet light with wavelengths from about 300 up to about 400 nm. The crystalline lens also absorbs a significant portion of the visible light at wavelengths of from 400 to about 450 nm, particularly as the lens ages and develops a yellow tint. In the aphakic eye, where there is no crystalline lens, light from 300 to 450 nm will be transmitted directly to the retina and the total spectrum of the light striking the retina in the aphakic eye will be different from that in the normal eye. As a consequence, aphakic patients are very sensitive to light in the ultraviolet range and may experience discomfort or color confusion when exposed to natural light or artificial light having high levels of ultraviolet wavelengths.

Intraocular lenses and hard contact lenses are presently produced from methyl methacrylate polymers, which exhibit a combination of properties desirable for such products, particularly optical clarity, the capability of being cut and polished to specific optical powers and chemical inertness. UV absorbing lenses of poly(methyl methacrylate) (PMMA) are required to maintain the above-mentioned desirable properties, while achieving at least 85% absorption of light at 400 nm, based on a polymer film thickness of 1 mm (millimeter). In addition, light absorption above a wavelength of 450 nm should be minimal, in order to avoid excessive yellowing of the lens.

Soft contact and intraocular lenses may be fabricated of silicone or fluorocarbon polymers, or hydrogels such as polymers of hydroxyethyl methacrylate and N-vinylpyrrolidone.

The benzotriazole monomers described in US-A-4,528,311 are effective UV absorbers and they form chemically stable copolymers with, for example, methyl methacrylate. However, a concentration of about 3 percent of the monomer in the copolymer is required to provide the desired degree of absorption at 400 nm. Also, the absorption of such copolymers cute off sharply above 400 nm, so that very little light in the 400 to 450 nm range is absorbed. As a consequence, lenses of such PMMA copolymers do not demonstrate the same absorption characteristics as the natural lens of an elderly person, who is the most frequent recipient of an intraocular lens.

It is an object of this invention to provide a copolymer composition with improved UV absorption characteristics. It is a further object to provide a novel UV absorbing monomeric material which is copolymerizable with vinyl monomers and which may be incorporated in silicone polymers. A still further object is to provide a new composition of matter which, when copolymerized at low concentrations with other monomers, effectively absorbs at least 85% of incident light below 420 nm at a 1 mm polymer film thickness. These and other objects of the present invention will be apparent from the ensuing description and claims.

The novel 2-(2′-hydroxyphenyl)-5(6)-(acryloyloxyalkoxy)-benzotriazoles provided by the invention are represented by Formula I :

I

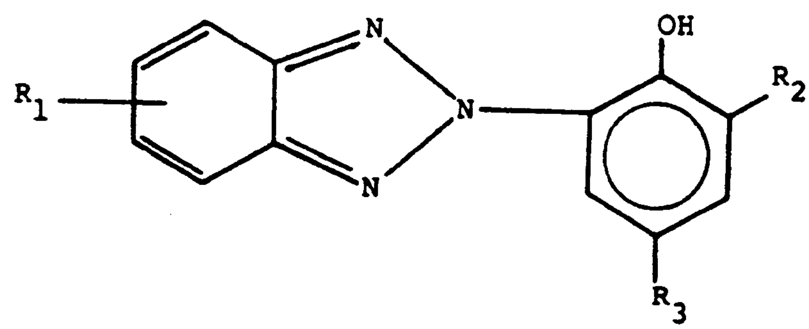

wherein $R_2$ represents hydrogen or alkyl of from 1 to 6 carbon atoms, $R_3$ represents hydrogen, halogen, alkyl of 1 to 5 carbon atoms, or alkoxy of 1 to 8 carbon atoms and $R_1$ represents a group of the formula :

$$\text{II} \quad -O-R'-O-CO-C(R'')=CH_2$$

wherein R′ is $C_2$ to $C_{10}$ alkylene, which may be straight chain or branched, and R″ represents hydrogen, methyl or halomethyl.

The above defined benzotriazoles are copolymerizable with vinyl monomers such as methyl methacrylate to yield polymers that are stabilized against degradation by UV radiation and which, when optically clear, are particularly useful in the preparation of intraocular, contact and spectacle lenses. From 0.01 to 20% by weight and preferably from 0.1 to 10% by weight of the benzotriazole compound is incorporated in the copolymer, the minimum effective amount for 85% absorption at 420 nm and 1 mm film thickness depending upon the particular structure of the benzotriazole compound. High molecular weight homopolymers of the benzotriazole monomers may also be prepared and incorporated into a variety of organic materials to impart UV absorption properties thereto. The benzotriazoles of the invention may also be incorporated in silicone polymers by the reaction of the acrylic or methacrylic groups with Si-H moieties.

In the accompanying drawings :

Fig. 1 is a plot of transmittance versus wavelength for a polymer of the invention (Curve C), in comparison with a polymer of US-A-4716234 (Curve B) and a human lens (Curve A) ; and

Figs. 2a and 2b show a sequence of reactions that can be used to produce the benzotriazole monomers

of the invention from readily available, known compounds.

The benzotriazole monomers of the present invention are those compositions that are represented by Formula I :

I

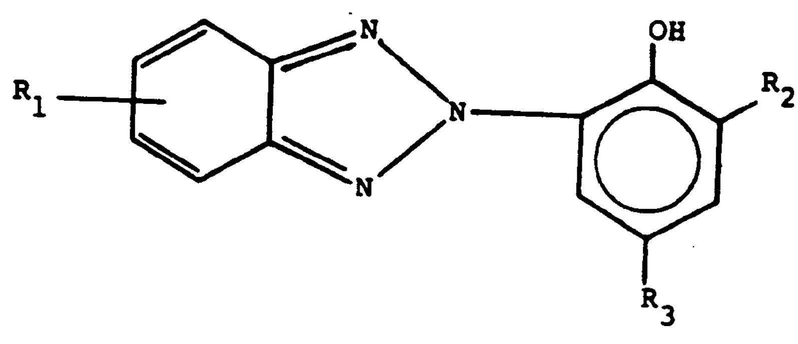

wherein $R_2$ represents hydrogen or alkyl of from 1 to 6 carbon atoms. $R_3$ represents hydrogen, halogen, alkyl of 1 to 5 carbon atoms or alkoxy of 1 to 8 carbon atoms and $R_1$ represents a group of the formula :

$$\text{II} \quad -O-R'-O-CO-C(R'')=CH_2$$

wherein R′ is $C_2$ to $C_{10}$ alkylene, which may be straight chain or branched, and R" represents hydrogen, methyl or halomethyl.

Particularly preferred compositions within the scope of Formula I are those wherein $R_1$ is in the 5 position and wherein R′ in $R_1$ is ethylene or propylene and R″ is methyl, wherein $R_2$ is t-butyl and $R_3$ is methoxy. A particularly preferred benzotriazole monomer of the invention is 2-(3′-t-butyl-2′-hydroxy-5′-methoxyphenyl)-5-(3″-methacryloyloxypropoxy)benzotriazole.

The preparation of this particularly preferred benzotriazole monomer is illustrated in the Examples below

A summary of the sequence of reactions that is displayed in Fig. 2a and 2b, and which can be employed to prepare the benzotriazole monomers of the invention, is the following :

4-Aminophenol (I) is reacted with a haloalkanol to produce a hydroxyalkoxy-aniline (II), which is then acetylated with a mixture of acetic anhydride and acetic acid to produce an acetoxyalkoxy-acetanilide (III). The reaction is here illustrated with 3-chloropropanol as the chloroalkanol. Other haloalkanols having from 2 to 10 carbon atoms may be employed. Specific illustrative examples of such other haloalkanols include 2-chloroethanol and 4-chlorobutanol.

III is nitrated with nitric acid and then hydrolyzed with aqueous alkali to produce a hydoxyalkoxy-2-nitroaniline (V).

V is diazotized by reaction with HONO (which may be derived from an acidified nitrite salt) and is then reacted with a substituted phenol, such as 2-t-butyl-4-methoxyphenol (which may be obtained from commercial t-butyl-hydroxyanisole), to produce an azo compound VI. The azo compound VI is then cyclized and reduced to produce a hydroxyalkoxy-benzotriazole VII. In place of the 2-t-butyl-4-methoxyphenol, there may be employed other compounds, such as 4-methoxyphenol, 2-methyl-4-methoxyphenol, 2-t-butyl-4-methylphenol, 2-methyl-4-chlorophenol and 2-t-butyl-4-chlorophenol.

The hydroxyalkoxy-benzotriazole VII, is then esterified by acrylic or methacrylic acid to produce a monomer of the invention VIII.

The following experiments illustrate this sequence of reactions :

EXAMPLE 1

Synthesis of 4-(3′-Acetoxypropoxy)-acetanilide (III)

A 3000 mL (milliliter), J-neck flask fitted with mechanical stirring, an inert gas inlet-topped reflux condenser and a thermocouple was charged with potassium hydroxide (70.4 g (grams), 1.1 mol, 88% pure pellets), METHYL CELLOSOLVE (1250 mL), 4-aminophenol (I) (109.1 g, 1.0 mol) and 3-chloropropanol (113.5 g, 1.2 mol). The mixture was heated to 100°C, maintained at that temperature for 3 hours, heated to the reflux temperature (about 118°C) and maintained at that temperature for 3 hours. The mixture was allowed to cool, kith stirring, overnight, was filtered to remove potassium chloride and was then stripped on a rotory evaporator to

an oil. The oil was taken up with acetic acid (300 mL) and was transferred to a 2000 mL, 3-neck flask fitted with mechanical stirring, an inert gas inlet-topped reflux condenser and a thermocouple. The solution was cooled to 0°C, then acetic anhydride (280 mL. 3 mol) was added over 100 minutes. The mixture was heated to 100°C, maintained there for 3.5 hours and then allowed to cool slowly to 40°C. The mixture was filtered and then stripped on a rotory evaporator (mechanical pump, 70°C) to a semi-solid (314 g). The semi-solid was dissolved in hot toluene (1000 mL). The toluene solution was filtered, washed first with 2% aqueous potassium hydroxide (400 mL) and then with water (500 mL). The toluene solution was dried over anhydrous sodium sulfate, filtered and then cooled to −4°C for 30 minutes. The solid precipitate was isolated by filtration and dried in vacuo to yield 113.2 g of III (76% pure by g.c.). The postulated structure was confirmed by IR and $^1$H NMR analyses.

EXAMPLE 2

Synthesis of 4-(3-Hydroxypropoxy)-2-nitroaniline (V)

A 1000 mL Erlenmeyer flask fitted with magnetic stirring and a thermocouple was charged with III (25.2 g, 0.10 mol), acetic anhydride (32.1 g. 0.31 mol) and methylene chloride (50 mL). The mixture was stirred at ambient temperature for 15 minutes to effect solution and was then cooled to −3°C in an ice-salt bath. Nitric acid (19.7 g, 0.22 mol, d = 1.41, 70% acid) was added in one portion. When the exothermic reaction was complete (maximum temperature 8.5°C), the cooling bath was removed. The temperature of the reaction mixture increased to 39.5°C, then slowly decreased over the next 60 minutes. A solution of potassium hydroxide (65.0 g, 1.02 mol of 88% base) in water (435 mL) was added. The dark mixture was heated to distill off volatile components. The final overhead temperature was 96°C after approximately 2.2 hours. The resulting mixture (pH = 13) was chilled in an ice bath to initiate crystallization and was then stored at 0°C overnight. The solid precipitate was isolated by filtration, dissolved in hot methanol (250 mL) and then water (1800 mL) was added. The mixture was heated to distill off moat of the methanol (overhead temperature 79°C), then chilled in an ice-bath to initiate crystallization. The mixture was stored at 0°C overnight. The resulting crystalline solid was isolated by filtration. The red-orange crystals were dried in vacuo (yield 13.0 g, mp 84.5-85.5°C). The postulated structure of V was confirmed by IR and $^1$H NMR analyses.

EXAMPLE 3

Separation of 2-tert-butyl-4-methoxyphenol from commercial BHA

Commercial BHA (butylated hydroxyanisole) is a mixture of 2-t-butyl-4-methoxyphenol (about 80%) and 3-t-butyl-4-methoxyphenol (about 20%). The isomers can be separated by exploiting the increased acidity of the phenolic OH non-adjacent to the t-butyl group in 3-t-butyl-4-methoxyphenol.

Commercial BHA (615 g) was dissolved in toluene (2000 mL), washed sequentially with 10% aqueous potassium hydroxide solution (3 × 1500 mL), water (1000 mL, made acidic with hydrochloric acid), water (1000 mL, final pH about 7), then dried over anhydrous sodium sulfate. The toluene solution was stripped to a solid (478 g) on a rotory evaporator. The solid was dried in vacuo to yield 2-t-butyl-4-methoxyphenol 93.2% pure (mp 59-69°C).

EXAMPLE 4

Synthesis of 2-(3′-t-butyl-2′-hydroxy-5′-methoxyphenyl)-5-(3″-hydroxypropoxy)benzotriazole (VII)

A 50 mL Erlenmeyer flask fitted with magnetic stirring was charged with V (2.3 g, 0.011 mol) and hydrochloric acid (3.5 mL. 0.035 mol). The mixture was stirred 5 minutes to effect solution, water (1.5 mL) was added and the solution was cooled to 0°C in an ice-salt bath. A solution of sodium nitrite (0.8 g, 0.012 mol) in water (2 mL) was added over 30 minutes and the resulting mixture was stirred at 0°C for 40 minutes. Sulfamic acid (0.2 g) was added to destroy excess nitrous acid (negative starch/iodide test). The solution was filtered and maintained at 0°C.

Meanwhile, a 250 mL, 3-neck flask fitted with mechanical stirring and a thermocouple was charged with 2-t-butyl-4-methoxyphenol (2.0 g, 0.011 mol), water (30 mL) and potassium hydroxide (2.6 g of 88% pure base, 0.04 mol). The mixture was heated to effect solution and then cooled to 0°C in an ice-salt bath. The clear diazonium ion solution from above was added dropwise over 10 minutes. The mixture was then stirred at 0°C for an additional 10 minutes (pH = 13). Hydrochloric acid (1N, 15 mL) was added (pH = 0) to precipitate the

intermediate azo compound, 2-t-butyl-6-(4′-(3″-hydroxypropoxy)-2′-nitrophenylazo]-4-methoxyphenol (VI).

The aqueous solution was removed from VI by suction. Reagent grade ethanol (50 mL) was added and the mixture was stirred to effect solution. Then a solution of sodium hydroxide (2.6 g, 0.07 mol), glucose (4.0 g, 0.02 mol) and water (30 mL) was added over 20 minutes. The solution was stirred at ambient temperature over a weekend. Zinc dust (10.0 g, activated with hydrochloric acid) was added, the mixture was stirred at ambient temperature for 2 hours and then heated to 65°C for 3 hours. The zinc was removed by filtration, the filtrate was acidified with hydrochloric acid (50 mL 1N) (pH = 1) and then extracted with methylene chloride (2 × mL). The methylene chloride extract was washed with water (3 × 40 mL, final wash pH = 7), dried over anhydrous sodium sulfate and stripped to a semi-solid (4.3 g) on a rotory evaporator. The semi-solid was passed through an alumina column (3 × 15 cm) with methylene chloride. The solution was stripped to oil (3.4 g) on a rotory evaporator. The oil was dissolved in hot heptane (125 mL). The resulting clear, yellow solution was allowed to cool slowly to ambient temperature and then stored at 0°C. The crystalline precipitate was isolated by filtration and dried in vacuo to yield 1.4 g of VII (mp 115-119°C). The postulated structure of VII was confirmed by IR and $^1$H NMR analyses.

## EXAMPLE 5

Synthesis of 2-(3′-t-butyl-2′-hydroxy-5′-methoxyphenyl)-5-(3″-methacryloyloxypropoxy)benzotriazole (VIII)

A 50 mL, round bottom flask fitted with magnetic stirring was charged with VII (0.8 g, 2 mmol), cyclohexane (25 mL), hydroquinone (0.01 g), p-toluenesulfonic acid (0.1 g, 0.53 m mol) and methacrylic acid (0.4 ml, 4.7 mmol). A distillation head fitted with an inert gas inlet-topped reflux condenser was attached to the flask. The apparatus was arranged so that condensate passed through a column of sand (20 g) and anhydrous sodium sulfate (10 g) before returning to the reaction flask. The mixture was heated to the reflux temperature and maintained at that temperature for 18 hours. The mixture was cooled and then methylene chloride (15 mL) and saturated aqueous sodium bicarbonate (15 mL) were added and stirred. The methylene chloride layer was separated, washed with water (1 × 40 mL, wash pH = 7), cyclohexane (150 mL) added and the solution was heated to distill off methylene chloride (overhead temperature 78.6°C, final volume 15 mL). The solution was cooled to ambient temperature and then chilled (about 10°C) with stirring for 2 hours. The precipitate was isolated by filtration and dried in vacuo to yield 0.7 g of VIII (mp 103-105°C). The postulated structure of VIII was confirmed by IR and $^1$H NMR analyses.

The following example illustrates the preparation of an ultraviolet absorbing polymer from the monomer of Example 5.

## EXAMPLE 6

### Preparation of Polymer

A test tube was charged with 0.30 g of VIII, 1.00 g of ethyl acrylate, 8.70 g of methyl methacrylate, 0.060 g of stearic acid, 0.0125 g of lauroyl peroxide and 56 microliters of 1-dodecanethiol. After all solid ingredients were dissolved, argon gas was bubbled into the mixture for about 20 seconds to remove oxygen. The solution was then filtered through a 0.2 μm polytetrafluoroethylene (PTFE) membrane and was placed in a Pyrex polymerization tube under an argon stream. The tube was closed with a PTFE-coated cap and was placed in a 60°C water bath for 18 hours. The resulting rod was post-polymerized at 110°C for 6 hours and was pressed into a 1-mm-thick film at about 200°C. The film showed transmittance of 0.931 at 700 nm, 0.155 at 430 nm and 0.0008 at 400 nm. The GC and HPLC analyses of the post polymerized rod showed residual monomers : 0.19% of ethyl acrylate : 0.21% of methyl methacrylate and 0.09% of VIII monomer.

The transmittance curves of the polymer of Example 6, above (a 1.16 mm thick film), identified as "C", a 1.02 mm thick film of a polymer of US-A-4716234, identified as "B", and the human crystalline lens of a person aged 50-60 years, identified as "A", are shown in Figure 1.

For optical uses, particularly contact lenses and intraocular lenses for aphakic patients, strong absorption of light up to about 420 nm is desired. In the case of the compound of Example 5, from 0.01 to 20 percent concentration, and particularly from 0.1 to 10 percent concentration, is desirable for such optical applications.

The benzotriazoles of the invention may be copolymerized with any of a number of unsaturated monomers to provide polymeric compositions having desirable UV stabilizing and absorbing characteristics. Alternatively, homopolymers or copolymers of the benzotriazoles of the invention may be utilized as additives to a wide variety of organic polymers to provide UV absorption properties. Representative of the polymers and copolymers useful in conjunction with the benzotriazole monomers and polymers of the invention are :

a. Polymers which are derived from mono- or diolefins, e.g. polyethylene, which can optionally be crosslinked, polypropylene, polyisobutylene, polymethylbutene-1, polymethylpentene-1, polyisoprene and polybutadiene ;
b. Mixtures of the homopolymers cited under (a), for example mixtures of polypropylene and polyethylene, polypropylene and polybutene-1 and polypropylene and polyisobutylene :
c. Copolymers of the monomers based on the homopolymers cited under (a), for example ethylene/propylene copolymers, propylene/butene-1 copolymers, propylene/isobutylene copolymers, ethylene/butene-1 copolymers, as well as terpolymers of ethylene and propylene with a diene, for example hexadiene, dicyclopentadiene or ethylidene norbornene, and copolymers of alpha-olefins, e.g. ethylene with acrylic or methacrylic acid ;
d. Polystyrene ;
e. Copolymers of styrene or of alpha-methylstyrene, for example styrene/butadiene copolymers, styrene/acrylonitrile copolymers, styrene/acrylonitrile/methacrylate copolymers, styrene/acrylonitrile copolymers modified with acrylic ester polymers to provide impact strength as well as block copolymers, e.g. styrene/butadiene/styrene block copolymers ;
f. Graft copolymers of styrene, for example the graft polymer of styrene to polybutadiene, the graft polymer of styrene with acrylonitrile to polybutadiene as well as mixtures thereof with the copolymers cited under (e), commonly referred to as acrylonitrile/butadiene/styrene or ABS plastics ;
g. Silicone polymers such as the soft hydrophilic polysiloxanes disclosed in US-A-4,259,467 and the hard polyorganosiloxanes disclosed in US-A-4,355,147 ;
h. Halogen-containing vinyl polymers, for example polyvinyl chloride, polyvinylidene chloride, polyvinyl fluoride, polychloroprene, chlorinated rubbers, vinyl chloride/vinylidene chloride copolymers, vinyl chloride/vinyl acetate copolymers and vinylidene chloride/vinyl acetate copolymers ;
i. Polymers which are derived from alpha, beta-unsaturated acids and derivatives thereof, polyacrylates and polymethacrylates, poly(hydroxyethyl methacrylate), polyacrylic amides and polyacrylonitrile. The instant compounds are advantageously used in heat-curable acrylic resin lacquers which are composed of a copolymer of acrylic acid and one or more of its derivatives, and a melanine-formaldehyde resin ;
j. Polymers which are derived from unsaturated alcohols and amines and from the acyl derivatives thereof or acetals, for example polyvinyl alcohol, polyvinyl acetate, polyvinyl stearate, polyvinyl benzoate, polyvinyl maleate, polyvinyl butyral, polyallyl phthalate, polyallyl melamine and copolymers thereof with other vinyl compounds, for example ethylene/vinyl acetate copolymers ;
k. Homopolymers and copolymers which are derived from epoxides, for example polyethylene oxide or the polymers which are derived from bis-glycidyl ethers ;
l. Polyacetals, for example polyoxymethylene, as well as polyoxymethylenes which contain ethylene oxide as comonomer ;
m. Polyalkylene oxides, for example polyoxyethylene, polypropylene oxide or polybutylene oxide ;
n. Polyphenylene oxides ;
o. Polyurethanes and polyureas, such as in urethane coatings ;
p. Polycarbonates ;
q. Polysulfones ;
r. Polyamides and copolyamides which are derived from diamines and dicarboxylic acids and/or from aminocarboxylic acids or the corresponding lactams, for example polyamide 6, polyamide 6/6, polyamide 6/10, polyamide 11, polyamide 12, n-vinyl lactams and poly-m-phenylene-isophthalamide ;
s. Polyesters which are derived from dicarboxylic acids and dialcohols and/or from hydroxycarboxylic acids or the corresponding lactones, for example polyethylene glycol terephthalate, poly-1,4-dimethylolcyclohexane terephthalate ;
t. Crosslinked polymers which are derived from aldehydes on the one hand and from phenols, ureas and melamine on the other, for example phenol/formaldehyde, urea/formaldehyde and melamine/formaldehyde resins ;
u. Alkyd resins, for example glycerol/phthalic acid resins and mixtures thereof with melamine/formaldehyde resins ;
v. Unsaturated polyester resins which are derived from copolyesters of saturated and unsaturated dicarboxylic acids with polyhydric alcohols, as well as from vinyl compounds such as styrene as crosslinking agents and also the halogen-containing, flame-resistant modifications thereof ; and
w. Natural polymers, for example cellulose, rubber, as well as the chemically modified homologous derivatives thereof, for example cellulose acetates, cellulose propionates and cellulose butyrates, and the cellulose ethers, for example methyl cellulose.

Particularly useful compositions are copolymers comprising from 0.01 to 20% by weight of benzotriazoles

of the invention with other ethylenically unsaturated materials such as styrene, methylstyrene, vinylsiloxanes, acrylates, methacrylates, acrylamide, acrylonitrile, methacrylonitrile, vinyl acetate, vinylidene chloride, vinyl chloride, vinyl fluoride, vinyl lactams, ethylene, propylene and mixtures thereof.

The homopolymers and copolymers of the benzotriazoles of the invention find wide application in formulating UV absorbing plastics and other organic materials wherever such materials are exposed to UV radiation from either natural or artificial sources. In addition to the medical use in intraocular and contact lenses described above, the materials of the present invention are useful in many industrial applications such as in solar energy collectors, polymeric coatings, transparent plastic films, fluorescent light diffusers, packaging materials, vinyl window coverings, automobile paints and interior coverings, epoxies and fiberglass constructions. Many other applications will be readily apparent to those familiar with this art as a result of reading the preceeding specification.

## Claims

1. A benzotriazole compound of the formula :

I

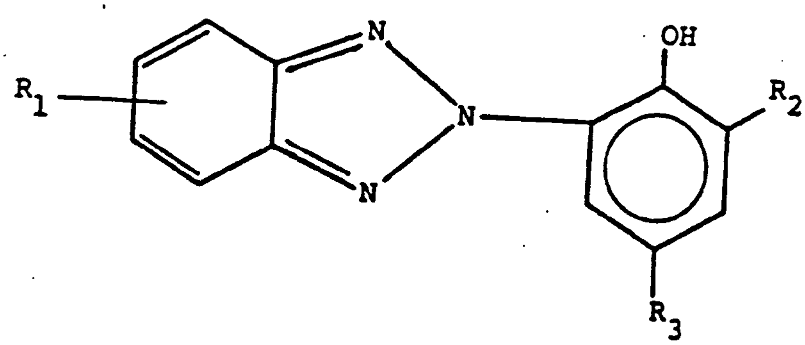

wherein $R_2$ represents hydrogen or alkyl of from 1 to 6 carbon atoms, $R_3$ represents hydrogen, halogen, alkyl of 1 to 5 carbon atoms or alkoxy of 1 to 8 carbon atoms, and $R_1$ represents a group of the formula :

$$\text{II} \qquad -O-R'-O-CO-C(R'')=CH_2$$

wherein R′ is $C_2$ to $C_{10}$ alkylene, which may be straight chain or branched, and R″ represents hydrogen, methyl or halomethyl.

2. The compound of claim 1 wherein $R_2$ is t-butyl.

3. The compound of claim 1 or Claim 2 wherein $R_3$ is methoxy.

4. The compound of claim 1 wherein said compound is 2-(3′-t-butyl-2′-hydroxy-5′-methoxyphenyl)-5-(3″-methacryloyloxypropoxy)benzotriazole.

5. An ultraviolet absorbing composition comprising a polymer of the compound of any one of claims 1 to 4.

6. An ultraviolet absorbing composition comprising a copolymer of an ethylenically unsaturated monomer and an effective amount of a benzotriazole compound of any one of claims 1 to 5.

7. The composition of claim 6 wherein said ethylenically unsaturated monomer is a styrene, methylstyrene, vinylsiloxane, acrylate, methacrylate, acrylamide, acrylonitrile, methacrylonitrile, vinyl acetate, vinylidene chloride, vinyl chloride, vinyl lactam, ethylene, propylene or a mixture thereof.

8. The composition of claim 6 or claim 7 wherein said ethylenically unsaturated monomer comprises hydroxyethyl methacrylate.

9. The composition of claim 6 or claim 7 wherein said ethylenically unsaturated monomer comprises methylmethacrylate.

10. The composition of any one of claims 6 to 9 wherein said benzotriazole compound comprises from 0.01 to 20% by weight of said composition.

11. The composition of any one of claims 6 to 10 wherein said benzotriazole compound comprises from 0.05 to 10.0% by weight of said composition.

12. An ultraviolet absorbing composition comprising an optically clear polymeric material and from 0.01 to

20% by weight of a benzotriazole compound of any one of claims 1 to 5.

13. The composition of claim 12 wherein said polymeric material is a polyvinyl halide, polyacrylate, polystyrene, polyvinylidene halide, polycarbonate or acrylonitrile-butadiene-styrene terpolymer.

14. The composition of claim 12 or claim 13 wherein said benzotriazole compound is a homopolymer of claim 5.

15. An optical lens comprising an optically clear polymer composition of any one of claims 6 to 14.

16. A lens of claim 15 which is a contact lens, intraocular lens or spectacle lens.

17. A contact lens or intraocular lens of claim 15 comprising a hydrogel of said optically clear polymer composition.

18. A plastic film or coating material comprising the composition of any one of claims 6 to 14.

**Patentansprüche**

1. Benzotriazol der Formel :

(I) 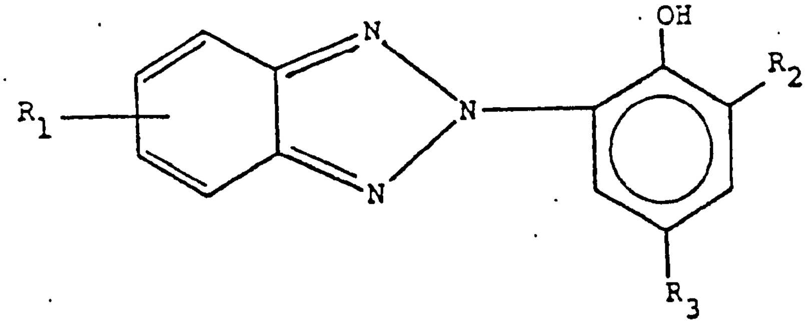

in der $R_2$ Wasserstoff oder Alkyl mit von 1 bis 6 Kohlenstoffatomen bedeutet, $R_3$ Wasserstoff, Halogen, Alkyl mit 1 bis 5 Kohlenstoffatomen oder Alkoxy mit 1 bis 8 Kohlenstoffatomen darstellt und $R_1$ eine Gruppe der Formel

(II) $-O-R'-O-CO-C(R'')=CH_2$

bedeutet, in der R′ $C_2$- bis $C_{10}$-Alkylen ist, das geradkettig oder verzweigt sein kann, und R" Wasserstoff, Methyl oder Halogenmethyl darstellt.

2. Verbindung nach Anspruch 1, in der $R_2$ t-Butyl ist.

3. Verbindung nach Anspruch 1 oder 2, in der $R_3$ Methoxy ist.

4. Verbindung nach Anspruch 1, die 2-(3′-t-Butyl-2′-hydroxy-5′-methoxyphenyl)-5-(3″-methacryloyl-oxypropoxy)-benzotriazol ist.

5. UV-absorbierende Zusammensetzung, enthaltend ein Polymer einer Verbindung nach einem der Ansprüche 1 bis 4.

6. UV-absorbierende Zusammensetzung, enthaltend ein Polymer eines ethylenisch-ungesättigten Monomers and eine wirkungsvolle Menge eines Benzotriazols nach einem der Ansprüche 1 bis 5.

7. Zusammensetzung nach Anspruch 6, in der das ethylenisch-ungesättigte Monomer Styrol, Methylstyrol, Vinylsiloxan, Acrylat, Methacrylat, Acrylamid, Acrylnitril, Methacrylnitril, Vinylacetat, Vinylidenchlorid, Vinylchlorid, Vinyllactam, Ethylen, Propylen oder ein Gemisch dieser Verbindungen ist.

8. Zusammensetzung nach Anspruch 6 oder 7, in der das ethylenisch-ungesättigte Monomer Hydroxyethylmethacrylat enthält.

9. Zusammensetzung nach Anspruch 6 oder 7, in der das ethylenisch-ungesättigte Monomer Methylmethacrylat enthält.

10. Zusammensetzung nach einem der Ansprüche 6 bis 9, in der das Benzotriazol von 0,01 bis 20 Gew.-% der Zusammensetzung ausmacht.

11. Zusammensetzung nach einem der Ansprüche 6 bis 10, in der das Benzotriazol von 0,05 bis 10,0 Gew.-% der Zusammensetzung ausmacht.

12. UV-absorbierende Zusammensetzung, enthaltend ein optisch klares polymeres Material und von 0,01 bis 20 Gew.-% eines Benzotriazols nach einem der Ansprüche 1 bis 5.

13. Zusammensetzung nach Anspruch 12, in der das polymere Material ein Polyvinylhalogenid, Polyacrylat, Polystyrol, Polyvinylidenhalogenid, Polycarbonat oder Acrylnitril-Butadien-Styrol-Terpolymer ist.

14. Zusammensetzung nach Anspruch 12 oder 13, in der das genannte Benzotriazol ein Homopolymer nach Anspruch 5 ist.

15. Optische Linse, enthaltend eine optisch klare polymere Zusammensetzung nach einem der Ansprüche 6 bis 14.

16. Linse nach Anspruch 15 als Kontaktlinse, Intraokularlinse oder Brillenglas.

17. Kontaktlinse oder Intraokularlinse nach Anspruch 15, enthaltend ein Hydrogel der genannten optisch klaren polymeren Zusammensetzung.

18. Kunststoff-Film oder Beschichtungsmaterial, enthaltend die Zusammensetzung nach einem der Ansprüche 6 bis 14.

## Revendications

1. Benzotriazole de formule :

I

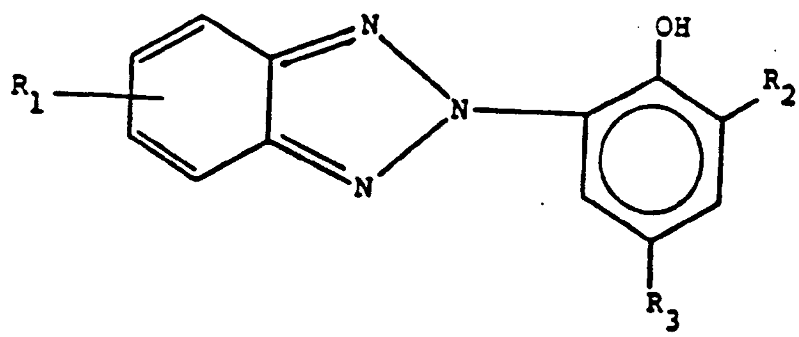

dans laquelle $R_2$ représente un hydrogène ou un alkyle de 1 à 6 atomes de carbone, $R_3$ représente un hydrogène, un halogène, un alkyle de 1 à 5 atomes de carbone ou un alcoxy de 1 à 8 atomes de carbone et $R_1$ représente un groupe de formule :

II $$-O-R'-O-CO-C(R'')=CH_2$$

dans laquelle R′ est un alkylène en $C_2$ à $C_{10}$ qui peut être à chaîne droite ou ramifiée et R″ représente un hydrogène, un méthyle ou un halogénométhyle.

2. Composé selon la revendication 1 où $R_2$ est un tert-butyle.

3. Composé selon la revendication 1 ou la revendication 2 où $R_3$ est un méthoxy.

4. Composé selon la revendication 1 qui est le 2-(3′-tert-butyl-2′-hydroxy-5′-méthoxyphényl)-5-(3″-méthacryloyloxypropoxy) benzotriazole.

5. Composition absorbant les rayons ultraviolets comprenant un polymère du composé de l'une quelconque des revendications 1 à 4.

6. Composition absorbant les rayons ultraviolets comprenant un copolymère d'un monomère à insaturation éthylénique et d'une quantité efficace d'un benzotriazole selon l'une quelconque des revendications 1 à 5.

7. Composition selon la revendication 6 dans laquelle ledit monomère à insaturation éthylénique est un styrène, méthylstyrène, vinylsiloxane, acrylate, méthacrylate, acrylamide, acrylonitrile, méthacrylonitrile, acétate de vinyle, chlorure du vinylidène, chlorure de vinyle, vinyllactame éthylène, propylène ou un de leurs mélanges.

8. Composition selon la revendication 6 ou la revendication 7 dans laquelle ledit monomère à insaturation éthylénique comprend du methacrylate d'hydroxyéthyle.

9. Composition selon la revendication 6 ou la revendication 7 dans laquelle ledit monomère à insaturation éthylénique comprend du méthacrylate de méthyle.

10. Composition selon l'une quelconque des revendications 6 à 9 dans laquelle ledit benzotriazole constitue de 0,01 à 20% du poids de ladite composition.

11. Composition selon l'une quelconque des revendications 6 à 10 dans laquelle ledit benzotriazole constitue de 0,05 à 10,0% du poids de ladite composition.

12. Composition absorbant les rayons ultraviolets comprenant une matière polymère optiquement transparente et de 0,01 à 20% en poids d'un benzotriazole selon l'une quelconque des revendications 1 à 5.

13. Composition selon la revendication 12 dans laquelle ladite matière polymère est un halogénure de polyvinyle, polyacrylate, polystyrène, halogenure de polyvinylidène, polycarbonate ou terpolymère d'acrylonitrile-butadiène-styrène.

14. Composition selon la revendication 12 ou la revendication 13 dans laquelle ledit benzotriazole est un homopolymère selon la revendication 5.

15. Lentille optique comprenant une composition de polymère optiquement transparente selon l'une quelconque des revendications 6 à 14.

16. Lentille selon la revendication 15 qui est une lentille de contact, une lentille intra-oculaire ou un verre de lunettes.

17. Lentille de contact ou lentille intra-oculaire selon la revendication 15 comprenant un hydrogel de ladite composition de polymère optiquement transparente.

18. Film ou matière de revêtement plastique comprenant la composition de l'une quelconque des revendications 6 à 14.

FIG-1

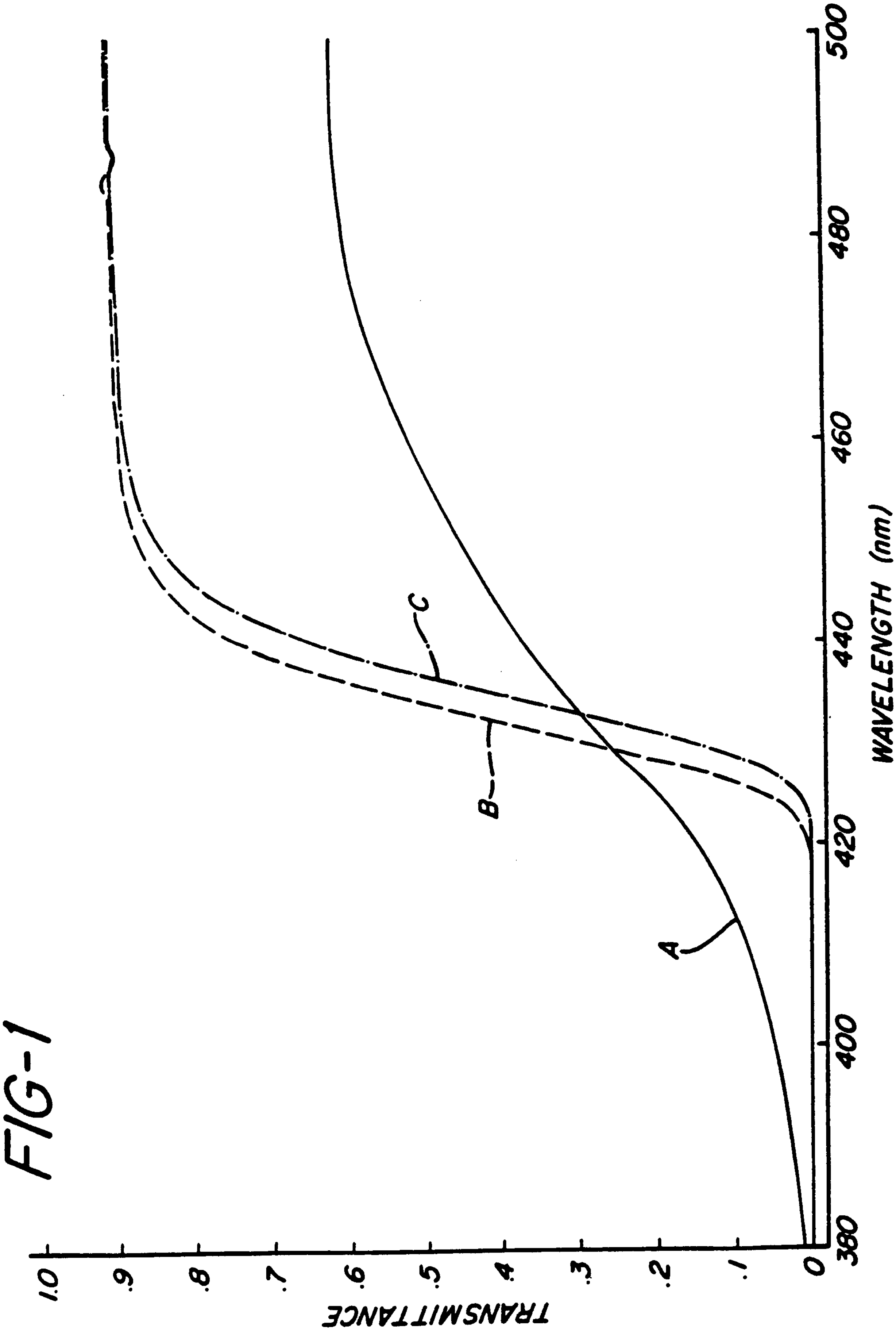
TRANSMITTANCE
1.0
.9
.8
.7
.6
.5
.4
.3
.2
.1
0
380
400
420
440
460
480
500
WAVELENGTH (nm)
A
B
C

FIG-2a

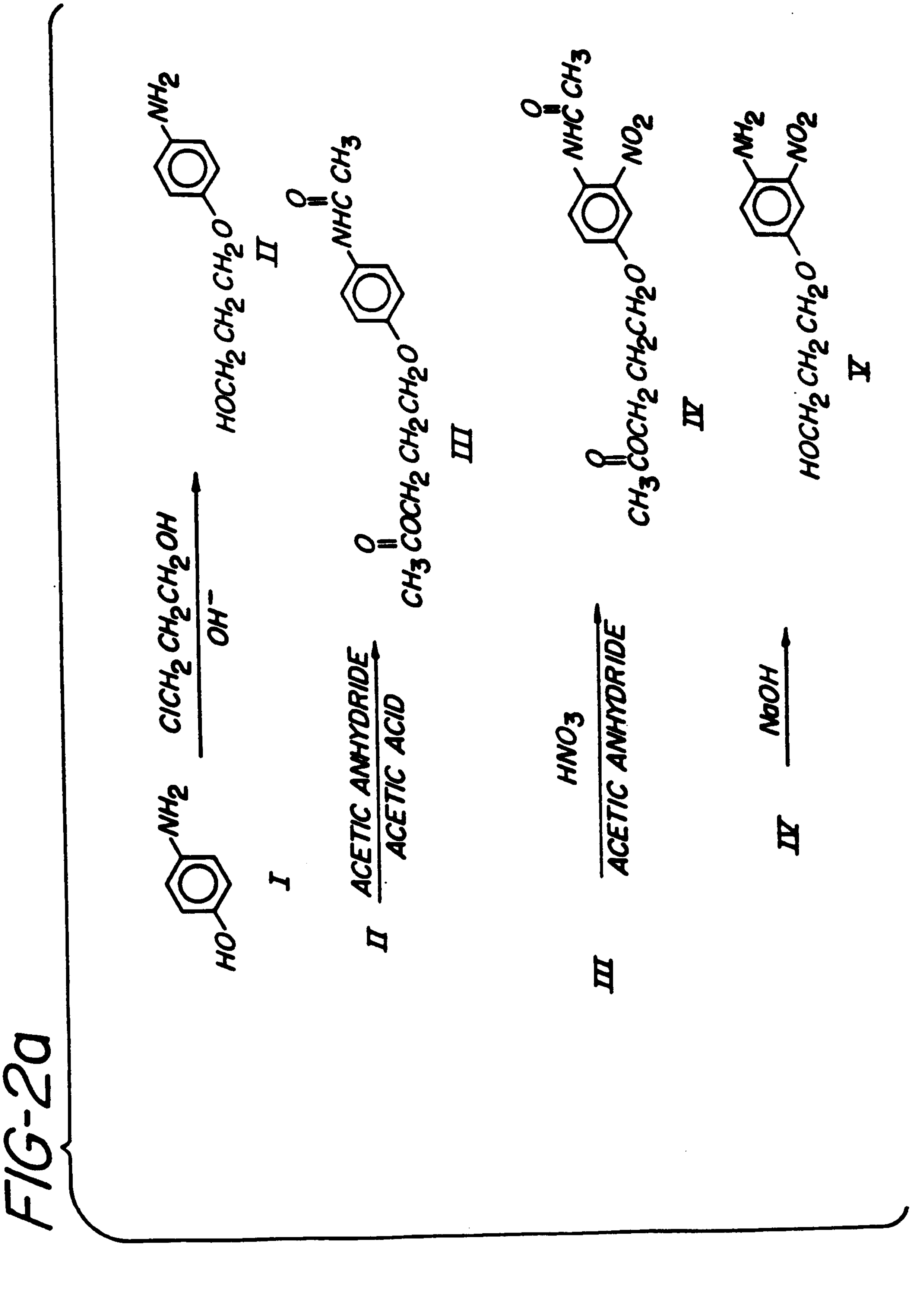
ClCH2CH2CH2OH
OH⁻
ACETIC ANHYDRIDE
ACETIC ACID
HNO3
ACETIC ANHYDRIDE
NaOH

FIG-2b

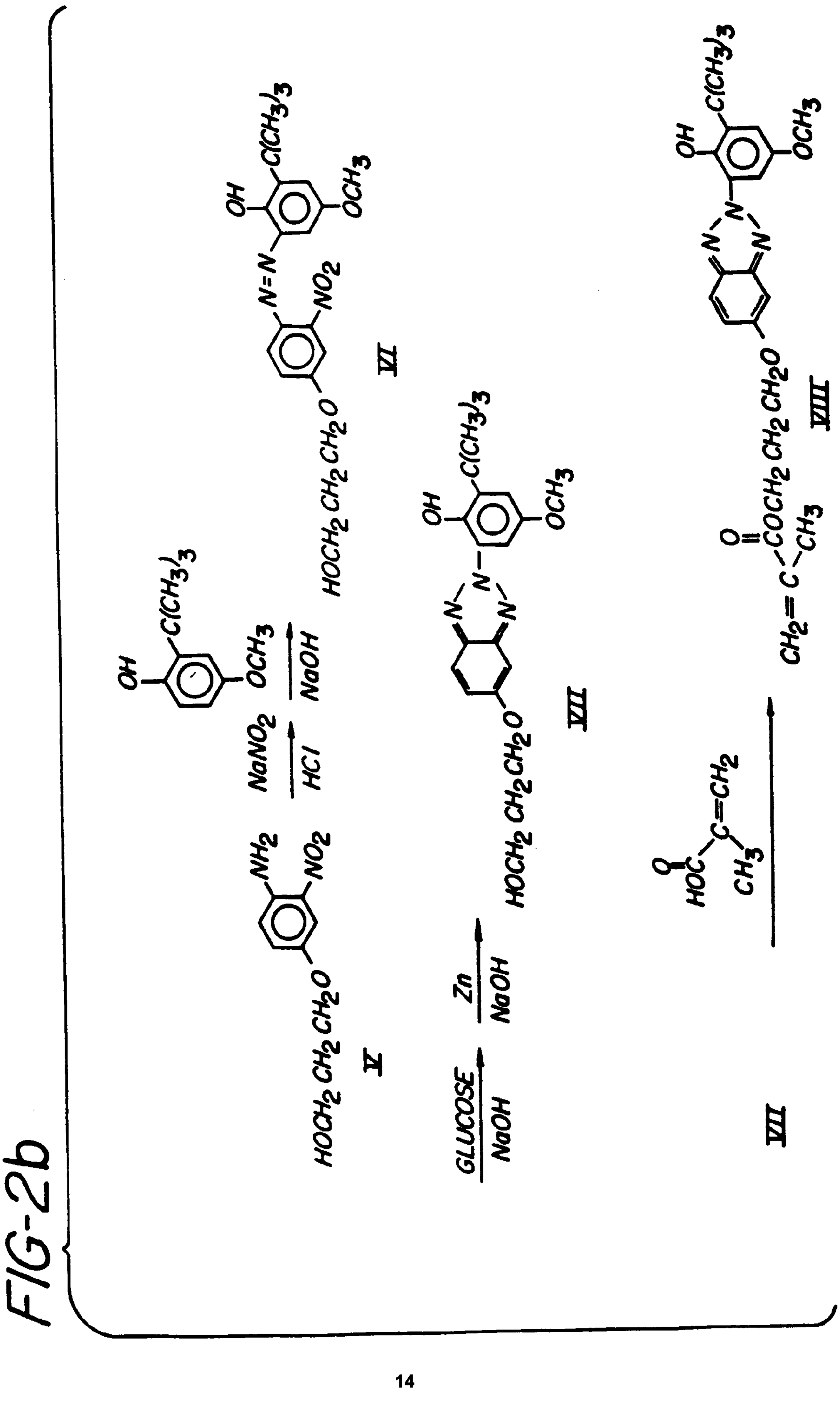


V

VI

VII

VII

VIII